## Europäisches Patentamt
## European Patent Office
## Office européen des brevets

(19)

(11) Numéro de publication: **0 042 808 B1**

(12) **FASCICULE DE BREVET EUROPEEN**

(45) Date de publication du fascicule du brevet:
**21.11.84**

(51) Int. Cl.³: **A 61 F 1/03**

(21) Numéro de dépôt: **81420090.3**

(22) Date de dépôt: **05.06.81**

(54) **Prothèse articulaire.**

(30) Priorité: **19.06.80 FR 8013915**

(43) Date de publication de la demande:
**30.12.81 Bulletin 81/52**

(45) Mention de la délivrance du brevet:
**21.11.84 Bulletin 84/47**

(84) Etats contractants désignés:
**AT BE CH DE GB IT LI LU NL SE**

(56) Documents cités:
**DE - A - 1 960 087**
**FR - A - 1 550 013**
**FR - A - 2 242 067**
**GB - A - 1 320 956**

**Le dossier contient des informations techniques présentées postérieurement au dépôt de la demande et ne figurant pas dans le présent fascicule.**

(73) Titulaire: **Gauthier, Georges, 85, cours Albert-Thomas, F-69003 Lyon (FR)**

(72) Inventeur: **Gauthier, Georges, 85, cours Albert-Thomas, F-69003 Lyon (FR)**

(74) Mandataire: **Maureau, Pierre, Cabinet GERMAIN & MAUREAU Le Britannia - Tour C 20, Boulevard E. Déruelle, F-69003 Lyon (FR)**

BUNDESDRUCKEREI BERLIN

# Description

La présente invention concerne une prothèse articulaire implantable du type révélé par le FR-A-2 242 067 et le DE-A-1 960 087, c'est-à-dire du type monobloc en matériau souple et élastique et comprenant entre deux prolongements destinés à être insérés dans les os de l'articulation concernée, une partie centrale présentant une zone élargie dans une direction transversale parallèle à l'articulation et amincie dans la direction transversale perpendiculaire à la précédente, la partie centrale présentant, de chaque côté de la zone amincie, un renflement arrondi dépassant dans toutes les directions les dimensions transversales du prolongement correspondant, englobant au moins toute la largeur de la zone articulaire amincie et formant une surface d'appui contre l'extrémité correspondante de l'os adjacent, le renflement situé du côté proximal étant plus volumineux que le renflement situé du côté distal, et les surfaces de ces renflements ne présentant pas d'arête ou autre siège d'amorce de rupture que ce soit au niveau de leur surface ou que ce soit au niveau des jonctions, soit avec la zone centrale articulaire, soit avec les prolongements proximal et distal.

Pour la clarté de la description, on appelle côté proximal de la prothèse le côté où le prolongement et le renflement sont les plus volumineux, c'est-à-dire le côté destiné généralement à être en rapport avec l'os le plus proche de la racine du membre. Inversement, on appelle côté distal, le côté opposé dont le renflement et le prolongement sont plus minces et qui est destiné à être en rapport avec l'os distal, c'est-à-dire le plus éloigné de la racine du membre. On supposera aussi que la prothèse est implantée horizontalement et la zone de flexion permettant un mouvement vertical, comme cela se fait dans les articulations métatarsophalangiennes, le pied étant à plat sur le sol. Il va de soi que toute autre disposition de la prothèse ne changerait rien à la nature de l'invention.

Ce type de prothèse est plus particulièrement destiné à remplacer les articulations des doigts ou des orteils. Mais, il peut aussi s'adapter à d'autres articulations telles que, par exemple, les articulations du tarse, du carpe, sternoclaviculaire ou acromioclaviculaire.

Or, l'articulation mobile normale a deux fonctions. La première fonction est le mouvement, ou modification des situations respectives entre les éléments articulaires. L'autre fonction est la transmission de forces mécaniques; en règle générale, des forces sont transmises d'un os à l'autre par une articulation, habituellement de façon plus ou moins perpendiculaire aux surfaces articulaires; mais, il existe aussi des appuis directs particulièrement importants sur certaines articulations, en particulier à travers la paume de la main ou la plante du pied.

Dans les prothèses actuelles précitées, les zones d'appui de la partie centrale contre chaque os sont généralement faibles et donc insuffisamment résistantes, de sorte que ces prothèses manquent de stabilité tant longitudinale que transversale, et notamment lorsque des forces axiales sont transmises par l'articulation, ce qui est sa fonction normale, ou lorsque des forces s'appliquent non dans le sens de la pliure articulaire, mais perpendiculairement à celle-ci, ce qui sollicite la stabilité de l'articulation, ou enfin lorsque s'effectuent des pressions directes à travers la plante du pied ou la paume de la main sur un des éléments articulaires.

Dans ces conditions, les prothèses actuelles en matériau souple et élastique, se déforment sous ce genre de pression et la prothèse articulaire, spécialement au niveau de la jonction entre le prolongement d'implantation et la zone articulaire, est soumise à des déformations importantes et des hyperpressions localisées qui sont facteur d'instabilité de la prothèse et d'usure prématurée de celle-ci. Ces prothèses sont donc prématurément détériorées et aussi inconfortables.

La présente invention vise à pallier ces inconvénients en fournissant des prothèses articulaires non seulement capables de réaliser les mouvements nécessaires au fonctionnement de l'articulation, mais présentant aussi une meilleure stabilité tant longitudinale que transversale et une meilleure longévité.

A cet effet, dans la prothèse qu'elle concerne et qui est du type précité, la surface d'appui formée par le renflement autour de la base du prolongement proximal est plane et forme un angle de 30 à 60° avec l'axe longitudinal de la prothèse, l'inclinaison étant telle que ladite surface d'appui orientée du côté opposé à la face plantaire ou palmaire de l'articulation remplacée, de manière à lui permettre de résister à une pression exercée sur cette face plantaire ou palmaire.

Lors de la pose de cette prothèse, la résection de l'extrémité correspondante de l'os proximal de l'articulation doit être exécutée avec une inclinaison correspondante de sorte que l'appui de l'épaulement se fasse sur la surface de résection de façon régulière. Ainsi, l'appui de l'épaulement précité contre l'extrémité réséquée de l'os proximal procure une stabilité souhaitée.

Suivant une autre caractéristique de l'invention, le renflement arrondi correspondant au côté proximal de l'articulation présente, du côté de la face plantaire ou de la face palmaire de cette articulation, une protubérance volumineuse.

La protubérance volumineuse rend cette prothèse particulièrement confortable et apte à l'appui à travers la paume de la main ou la plante du pied. Cette protubérance, volumineuse lors de l'appui, évite de gêner le fonctionnement des tendons fléchisseurs qui doivent passer sur cette face de l'articulation et qui sont élément principal de sa fonction.

Suivant encore une autre caractéristique de l'invention, du côté distal, côté où la surface ostéo-articulaire présente une conformation concave, le renflement arrondi correspondant est conformé à la forme de ladite surface concave. On obtient ainsi une large surface d'appui de la prothèse sur une portion articulaire ostéo-cartilagineuse distale, non réséquée. Cela permet d'améliorer la stabilité longitudinale; en outre, la pénétration partielle du renflement considéré dans la surface concave ostéo-cartilagineuse distale procure à ce côté de la prothèse une bonne stabilité transversale et verticale. Cette caractéristique présente aussi l'avantage d'éviter une résection de l'os distal dans lequel on devra seulement effectuer une perforation centrale pour implanter le prolongement distal de la prothèse; ceci limite les risques d'ossification périphérique autor de la prothèse, de ce côté au moins, puisque la portion distale de la prothèse sera en contact sur toute sa périphérie avec une zone ostéo-cartilagineuse non réséquée, moins susceptible de faire des ossifications qu'une surface osseuse cruentée. Enfin, la surface ostéo-cartilagineuse ainsi conservée est naturellement sans aspérité et ne sera donc pas traumatisante pour la prothèse.

De toute façon, l'invention sera bien comprise à l'aide de la description qui suit, en référence au dessin schématique annexé représentant, à titre d'exemple non limitatif, une forme d'exécution préférentielle de cette prothèse articulaire:

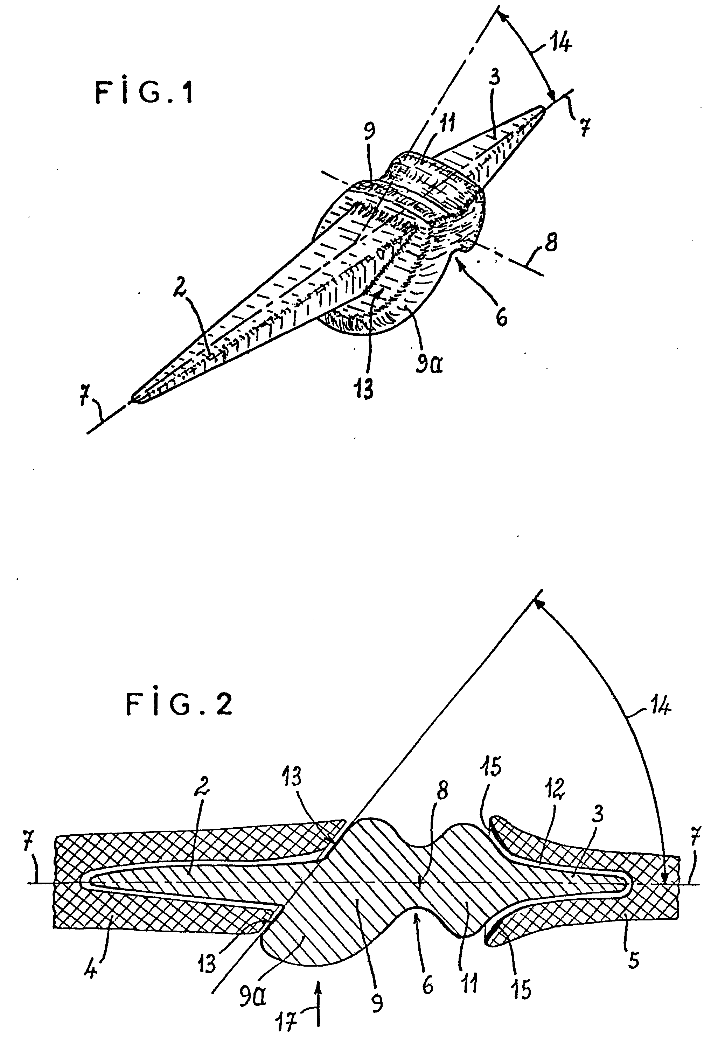

Fig. 1 en est une vue en perspective;

Fig. 2 en est une vue en coupe longitudinale.

Comme le montre le dessin, cette prothèse est du type réalisé en une seule pièce monobloc en matériau souple et élastique, tel qu'un élastomère de silicone biocompatible. Cette prothèse est du type comprenant, entre deux prolongements, l'un proximal 2 et l'autre distal 3, desinés à être insérés dans les os respectivement proximal 4 et distal 5, une zone 6 amincie dans le sens perpendiculaire au plan comprenant l'axe longitudinal 7 des prolongements 2 et 3 et l'axe d'articulation 8 et élargie dans le sens dudit axe d'articulation.

Comme le montre le dessin, de chaque côté de la zone amincie 6, ou zone articulaire, cette prothèse présente un renflement arrondi, respectivement 9 du côté proximal et 11 du côté distal. Il résulte de l'arrondi de ces deux renflements 9 et 11 que cette prothèse ne présente aucune arête susceptible de constituer le siège d'une amorce de rupture et qu'en outre leur volume procure à cette prothèse la résistance nécessaire à l'encontre des forces, notamment celles qui s'exercent longitudinalement, celles qui s'exercent verticalement dans l'appui direct à la paume de la main ou la plante de pied, celles qui s'exercent transversalement pour solliciter la stabilité de la prothèse.

Comme le montre le dessin, chaque renflement 9 ou 11 a des dimensions transversales et verticales dépassant largement les dimensions transversales et verticales du prolongement correspondant 2 ou 3. En outre, chaque renflement 9 ou 11 englobe toute la largeur de la zone articulaire amincie 6.

Suivant une réalisation préférentielle de l'invention, la face externe du renflement distal 11, tournée vers la face concave de l'articulation est conformée au profil de cette face concave. On obtient ainsi, une surface d'appui entre la surface ostéo-cartilagineuse distale 15 et le renflement distal 11 considéré. Il résulte de l'engagement partiel du renflement distal 11 dans l'extrémité concave de la surface ostéo-articulaire distale 15, une amélioration de la stabilité horizontale et verticale de l'articulation au niveau distal. Comme on le voit, l'implantation de cette prothèse, côté distal, n'exige que l'exécution de la perforation 12 nécessaire à l'engagement du prolongement 3, sans qu'il soit besoin de pratiquer une résection de l'extrémité de l'os 5. On diminue ainsi considérablement les risques de traumatisme de la portion distale de la prothèse par une surface de section osseuse et les risques d'ossification périphérique autor de la prothèse.

Du côté de l'os proximal 4, d'une part, le renflement proximal 9 présente à sa partie inférieure du côté de la face d'appui normale de l'articulation, une protubérance spécialement volumineuse 9a, et, d'autre part, l'épaulement 13 formé par le renflement proximal 9 et sa protubérance 9a autor de la base du prolongement proximal 2 a une inclinaison formant un angle 14 de l'ordre de 45 degrés par exemple, par rapport à l'axe longitudinal 7 des prolongements 2 et 3, cette inclinaison est orientée de manière que la face 13 soit tournée du côté opposé à la protubérance 9a, c'est-à-dire la face d'appui normal de l'articulation.

Naturellement, avant la mise en place de cette prothèse, l'extrémité correspondante de l'os proximal 4 doit être réséquée avec une inclinaison complémentaire.

On conçoit aisément qu'il en résulte que, lorsque sur l'articulation équipée de cette prothèse est appliquée par sa face d'appui une force 17, d'une part, la protubérance 9a reçoit la réaction qui résulte de cet appui sans traumatiser les tissus interposés, et, d'autre part, l'application de l'épaulement 13 contre l'extrémité correspondante réséquée de l'os proximal 4 contribue à procurer à cette prothèse du côté proximal de l'articulation, la stabilité désirée. En effet, la réaction de l'extrémité réséquée de l'os proximal 4 contre l'épaulement 13 s'oppose à tout déplacement de la prothèse dans le sens vertical, c'est-à-dire celui de la flèche 17.

Grâce à l'ensemble de ces caractéristiques, cette prothèse présente donc une excellente stabilité longitudinale, verticale, et transversale; elle est de plus particulièrement confortable aux appuis directs, plantaires ou palmaires.

**Revendications**

1. Prothèse articulaire implantable du type monobloc en matériau souple et élastique comprenant, entre deux prolongements proximal (2) et distal (3) destinés à être insérés dans les os proximal (4) et distal (5) de l'articulation concernée, une partie centrale présentant une zone (6) élargie suivant une direction transversale parallèle à l'axe d'articulation et amincie dans la direction transversale perpendiculaire à la précédente, la partie centrale présentant, de chaque côté de la zone amincie (6), un renflement arrondi (9) et (11) dépassant dans toutes les directions les dimensions transversales du prolongement correspondant (2) et (3), englobant au moins toute la largeur de la zone articulaire amincie (6) et formant une surface d'appui (13) contre l'extrémité correspondante de l'os adjacent, le renflement situé du côté proximal (9) étant plus volumineux que le renflement situé du côté distal (11), et les surfaces de ces renflements ne présentant pas d'arête ou autre siège d'amorce de rupture, que ce soit au niveau de leur surface ou que ce soit au niveau des jonctions, soit avec la zone centrale articulaire (6), soit avec les prolongements proximal (2) et distal (3), caractérisée ence que la surface d'appui (13) formé par le renflement (9) autour de la base du prolongement proximal (2) est plane et forme un angle (14) de 30 à 60° avec l'axe longitudinal (7) de la prothèse, l'inclinaison étant telle que ladite surface d'appui (13) est orientée du côté opposé à la face plantaire ou palmaire de l'articulation remplacée, de manière à lui permettre de résister à une pression exercée sur cette face plantaire ou palmaire.

2. Prothèse selon la revendication 1, caractérisée en ce que le renflement arrondi (9) correspondant au côté proximal de l'articulation présente, du côté de la face plantaire ou de la face palmaire de cette articulation, une protubérance (9a) volumineuse.

3. Prothèse selon la revendication 1 ou la revendication 2, caractérisée en ce que, du côté distal, c'est-à-dire du côté où l'os (5) présente une surface articulaire concave, le renflement arrondi correspondant (11) est conformé en profil de ladite surface articulaire concave.

## Patentansprüche

1. Implantierbare Gelenkprothese des einstükkigen Types aus biegsamem und elastischem Material, beinhaltend zwischen zwei naheliegenden (2) und fernliegenden (3), in die naheliegenden (4) und fernliegenden (5) Knochen des Gelenkes einzubringenden Verlängerungen einen zentralen Abschnitt, der gemäß einer Transversalrichtung parallel zur Gelenkachse eine verbreiterte Zone und in der Transversalrichtung senkrecht zur vorhergehenden eine verdünnte Zone aufweist, wobei der zentrale Abschnitt auf jeder Seite der verdünnten Zone (6) einen gerundeten Wulst (9 und 11) aufweist, der in allen Richtungen die Transversaldimensionen der entsprechenden Verlängerung (2 und 3) übersteigt und mindestens die gesamte Breite der verdünnten Gelenkzone (6) einschließt und eine Abstützfläche (13) gegenüber dem entsprechenden Ende des benachbarten Knochens bildet, wobei der auf der naheliegenden Seite liegende Wulst (9) voluminöser als der auf der fernliegenden Seite liegende Wulst (11) ist und die Oberflächen dieser Wulste keine Kante oder eine andere Bruchausgangsstelle haben, sei es im Bereich ihrer Oberflächen oder sei es im Bereich der Verbindungen entweder mit der zentralen Gelenkzone (6) oder mit den naheliegenden und fernliegenden Verlängerungen (2 und 3), dadurch gekennzeichnet, daß die von dem Wulst (9) um die Basis der naheliegenden Verlängerung (2) herum gebildete Abstützfläche (13) eben ist und einen Winkel (14) von 30—60° mit der Längsachse (7) der Prothese bildet, wobei die Neigung derart ist, daß die genannte Abstützfläche (13) auf die Seite gerichtet ist, die der Flußfläche oder Handfläche des ersetzten Gelenkes gegenüberliegt, derart, daß es einem auf diese Fußfläche oder Handfläche ausgeübten Druck widerstehen kann.

2. Prothese nach Anspruch 1, dadurch gekennzeichnet, daß der der naheliegenden Seite des Gelenkes entsprechende gerundete Wulst (9) auf Seiten der Handfläche oder der Fußfläche dieses Gelenkes eine voluminöse Ausstülpung (9a) aufweist.

3. Prothese nach Anspruch 1 oder 2, dadurch gekennzeichnet, daß auf der fernliegenden Seite, d. h. auf der Seite, auf der der Knochen (5) eine konkave Gelenkoberfläche hat, der entsprechende gerundete Wulst (11) dem Profil dieser konkaven Gelenkoberfläche entsprechend geformt ist.

## Claims

1. An implantable articular prosthesis of the type which is all in one piece and is made of a flexible and resilient material comprising, between a proximal extension (2) and a distal extension (3) which are to be inserted into the proximal bones (4) and distal bones (5) of the joint in question, a central part having an area (6) broadened in a transverse direction parallel to the axis of articulation and tapered in the transverse direction perpendicular to the preceding direction, the central part having, on each side of the tapered area (6), a rounded enlargement (9) and (11) exceeding, in all directions, the transverse dimensions of the corresponding extension (2) and (3), enclosing at least the entire width of the tapered articular area (6) and forming a bearing surface (13) against the corresponding end of the adjacent bone, the enlargement situated on the proximal side (9) being greater than the enlargement situated on the distal side (11), and the surface of these enlargements do not have any ridge or other centre for an incipient fracture, either on their surfaces or at the junctions, either with the central articular area (6) or with the proximal extension (2) and distal extension (3), characterized in that the bearing surface (13) formed by the enlargement (9) around the base of the proximal extension (2) is flat and forms an angle (14) of 30 to 60° with the longitudinal axis (7) of the prosthesis, the inclination being such that the said bearing surface (13) is directed, on the side opposite the plantar or palmar face of the replaced joint, so as to allow it to resist a pressure exerted on this plantar or palmar face.

2. A prosthesis according to claim 1, characterized in that the rounded enlargement (9) corresponding to the proximal side of the joint has, on the side of the plantar face or the palmar face of this joint, a large protuberance (9a).

3. A prosthesis according to claim 1 or claim 2, characterized in that, on the distal side, i. e. on the side where the bone (5) has a concave articular surface, the corresponding rounded enlargement (11) is shaped at the profile of the said concave articular surface.

FIG.1

FIG.2